# EUROPEAN PATENT SPECIFICATION

(11) **EP 0 923 382 B1**
(45) Date of publication and mention of the grant of the patent: **29.05.2002**
(21) Application number: 97935205.1
(22) Date of filing: 04.08.1997
(51) Int. Cl.: A61K 38/16, A23L 1/09, A23L 1/0526, A23L 1/053, A23L 1/0534, A23L 1/054, A23L 1/0524, A23L 1/0528, A23L 1/0532, A23L 1/212

(54) **COMPOSITIONS OF PLANT CARBOHYDRATES AS DIETARY SUPPLEMENTS**
ZUSAMMENSETZUNGEN AUF PFLANZLIICHER KOHLENHYDRATBASIS ALS NAHRUNGSERGÄNZUNGSSTOFFE
COMPOSITIONS A BASE D'HYDRATES DE CARBONE DE PLANTES COMME SUPPLEMENTS DIETETIQUES

(30) Priority: 09.08.1996 US 22467 P; 01.11.1996 US 30317 P; 24.07.1997 US 57017 P
(43) Date of publication of application: 23.06.1999
(60) Divisional application: 01123879.7
(73) Proprietor: Mannatech, Inc., Coppell, TX 75019 (US)
(72) Inventor: McANALLEY, Bill, H., Grand Prairie, TX 75052 (US); McDANIEL, H., Reginald, Mansfield, TX 76063 (US); MOORE, D., Eric, Richardson, TX 75080 (US); VENNUM, Eileen, P., Grand Prairie, TX 75050 (US); FIORETTI, William, C., Grapevine, TX 76051 (US)
(74) Representative: Hanna, Peter William Derek
(86) International application number: US9713379
(87) International publication number: WO9806418

(56) References cited:
- EP-A- 0 052 171
- WO-A-91/15214
- DE-A- 3 935 906
- US-A- 4 665 057
- US-A- 4 837 040
- US-A- 5 106 616
- US-A- 5 308 838
- US-A- 5 378 480
- PATENT ABSTRACTS OF JAPAN vol. 016, no. 054 (C-0909), 12 February 1992 (1992-02-12) & JP 03 255029 A (NISSHIN FLOUR MILLING CO LTD), 13 November 1991 (1991-11-13)
- PATENT ABSTRACTS OF JAPAN vol. 1996, no. 01, 31 January 1996 (1996-01-31) -& JP 07 242551 A (FUJISAWA PHARMACEUT CO LTD), 19 September 1995 (1995-09-19)
- DATABASE WPI Section Ch, Week 199833 Derwent Publications Ltd., London, GB; Class B03, AN 1998-385572 XP002120265 -& RU 2 097 041 C (CHEPURNOI I P), 27 November 1997 (1997-11-27)

## Description

### FIELD OF THE INVENTION

This invention pertains to the field of dietary supplements and nutritional support for promotion and maintenance of good health. More specifically, the invention relates to compositions of carbohydrates as dietary supplements that are essential for the production of correctly structured and, therefore, properly functioning glycoproteins.

### DESCRIPTION OF THE PRIOR ART AND OTHER INFORMATION

The term mucus was first used in the 1700s. By 1805, Bostok realized that mucus was composed of protein that differed from albumin and gelatin. In 1865, Eichwald showed that mucins contained carbohydrate moieties. In 1877, Hoppe-Seyler discovered that mucins were high in sialic acid content. In 1882, Landwehr showed that plant gums, a type of mucin, contain more than one monosaccharide. With the advent of more modem methods, these monosaccharides were isolated and characterized. In 1888. Harmarsten showed that the saccharides in mucins were joined by a covalent bond; Harmarsten was the first to use the term "glykoproteide" (or glycoprotein in English). Fischer and Leuchs discovered high concentrations of mannose in mucus in 1902. Hayworth, in 1939, discovered N-acetylglucosamine and Bierry discovered galactose in 1930. Meyer discovered fucose in 1958 (Gottschalk, Glycoproteins. 1972).

Proteins were originally thought to be the primary "communication" molecules of the body. The biotechnology revolution began as an attempt to create new drugs based upon proteins which are made up of various combinations of amino acids. However, since amino acids can only bind to each other through an amide bond, the number of secondary configurations possible with proteins is limited. Indeed, only one secondary configuration is possible per dipeptide.

However, many more functions are performed by the body than can be accounted for by the number of molecular configurations possible with proteins. Several years ago a theoretical mathematician calculated the number of configurations possible with proteins and discovered that another mechanism, yet unknown, had to be responsible for performing most of the communication functions of the body. It is now known that this mechanism involves carbohydrates.

In contrast to the simpler proteins, more molecular configurations are possible with the more complex carbohydrate molecule, e.g., a hexose has six chiral centers each of which has two isomeric forms and each of which has a hydroxyl group as a binding site for other molecules. Thus, while only 24 oligopeptide configurations are possible with four amino acids, more than 100,000 different oligosaccharide configurations are possible with four sugars (Stryer et al., Biochemistry, 1995; p 477).

Science has recently shown that glycoproteins play a key role in all cellular communication. Many of the cytokines, i.e. cellular messenger agents, do not function properly without an attached glycosyl moiety. The body hydrolyzes complex polysaccharides such as plant carbohydrates into various monosugars and restructures them into oligosaccharides that are then used by the body to build the glycoproteins required by cytokines for cellular communication and, thus, for good health.

With the advent of improved analytical techniques and more powerful computers, characterization of glycoproteins increased rapidly after the 1960s. By the mid 1980s, the mechanism of the orderly synthesis of glycoproteins in the endoplasmic reticulum and Golgi apparatus had been determined. The actual oligosaccharide conformations of many glycoproteins is now known.

Increasing interest in glycobiology has been precipitated by recent findings that cell surface carbohydrates are critically involved in cell adhesion and, thus, in cell-cell interaction. Specifically, three new mechanistic concepts have been discovered. First, structural studies in glycoproteins and glycolipids have revealed the existence of carbohydrates which are unique to certain cell types. This concept is crucial to understanding cell surface carbohydrates as cell-type specific recognition molecules.

A second concept was developed from new information regarding lectins, which have sugar-binding proteins. In the 1970s it was learned that glycoproteins were removed rapidly from the blood when their sialic acid, i.e. N-acetylneuraminic acid, containing branches were removed. Further studies revealed that this rapid clearance was caused by asialoglycoproteins binding to lectins that recognize terminal galactose. Once animal cells were known to have lectins, a large number of lectins were characterized, and a dedicated section in the amino acid sequence that is responsible for the carbohydrate recognition domain in the lectins was discovered. This discovery was critical to understanding carbohydrate-binding capability in cell-cell interactions. Thus, cellular communication was recognized at the molecular level.

The third concept resulted from studies regarding the isolation and characterization of the glycosyltransferases that form carbohydrates. These studies showed that carbohydrate moieties are usually built one by one, and each reaction is carried out by a glycosyltransferase that forms only a specific linkage. The advent of molecular biology in this field has enabled scientists to manipulate carbohydrate expression and study glycoprotein function.

Based on critical advances in this field, the most recent studies demonstrated that oligosaccharides uniquely present in leukocytes act as ligands for adhesive molecules in endothelia and platelets. When these adhesive molecules, known as selectins, were cloned, it was discovered that they contained carbohydrate recognition domains. Thus, studies on cell-type specific carbohydrates and animal lectins corroborated each other. Moreover, these studies were preceded by the findings that lymphocyte-endothelial interaction is dependent upon carbohydrates.

Given the above, research directed toward the synthesis of drugs that would correct malformation of glycoproteins on cell surfaces began. After the carbohydrate ligand sialyl-Le^{x} was identified, pharmaceutical companies soon synthesized it for therapeutic purposes. This line of research has since become much easier because enzymatic synthesis of carbohydrates is now possible thanks to the availability of glycosyltransferases generated by cloned cDNAs (Fukuda et al., Glycobiology, 1994).

The synthesis of all proteins and glycoproteins is controlled by somatic genes embodied in the chromosomes of a cell. The coding information expressed in nucleic acids (DNA) controls all cellular functions, including general body defense, regeneration, remodeling and healing. Though DNA provides the blueprint, the cellular components cannot be built correctly without the required building blocks. As discussed above, cytokines are key components used for intracellular instruction to carry out the body's vital functions. However, many cytokines do not function roperly without an attached glycosyl moiety.

Table 1 lists some of the known physiological functions served by glycoproteins. Table 2 lists some of the specific known functions that the oligosaccharide branches or chains of glycoproteins perform.

**Table 1.**

| **Some known functions served by glycoproteins:** | |
|---|---|
| Function | Glycoproteins |
| Structural molecule | Collagens |
| | |
| Lubricant and protective agent | Mucins |
| | |
| Transport molecule | Transferrin, ceruloplasmin |
| | |
| Immunologic molecule | Immunoglobulins, histocompatibility antigens |
| | |
| Hormone | Chorionic gonadotropin, thyroid-stimulating hormone (TSH) |
| | |
| Enzyme | Various, e.g., alkaline phosphatase |
| | |
| Cell attachment-recognition site | Various proteins involved in cell-cell (e.g., sperm-oocyte), virus-cell, bacterium- cell, and hormone-cell interactions |
| | |
| Interact with specific carbohydrates | Some lectins |

**Table 2.**

| **Some known functions of the oligosaccharide chains of glycoproteins:** |
|---|
| * Modulate physicochemical properties, e.g., solubility, viscosity, charge, and protein denaturation |
| * Protect against proteolysis from within and outside the cell |
| * Affect proteolytic processing of precursor proteins to smaller products |
| * Are involved in biologic activity, e.g., of human chorionic gonadotropin (hCG) |
| * Affect insertion of protein into membranes, intracellular protein migration, and protein sorting and secretion |
| * Affect embryonic development and differentiation |
| * Affect metabolism |
| * May affect sites of metastases selected by cancer cells |

In summary, various processes of the cell are regulated or affected by correctly structured and, therefore, properly functioning glycoproteins.

Despite the above discussed current scientific knowledge concerning the importance of glycoproteins to cell-cell communication and the importance of carbohydrates in the formation of glycoproteins, and despite the fact that diet is the source of a majority of carbohydrates, the fields of glycobiology and nutrition have never been adequately investigated together. Although current nutrition textbooks stress the importance of essential vitamins, minerals, proteins (amino acids) and fats in great detail, sugars are currently recognized only as a source of energy (Shils et al., 1994)--not as substances essential to glycoprotein production for good health. For example, Shils et al. disclose that the principal sources of dietary carbohydrates are: 1) maize, rice, wheat, and potato which yield starches comprising glucose; 2) sugar cane and beet sugar which yield fructose and glucose; and 3) milk which yields galactose and glucose (Shils et al., Modern Nutrition in Health and Disease, (1994)).

By way of contrast, Harper's Biochemistry (Murray et al., 1996) lists eight and Principles of Biochemistry, Vol II (Zubay et al., 1995) lists eleven monosaccharides commonly found in the oligosaccharide chains of cellular glycoproteins. Thus, of the approximate 200 monosaccharides found in nature, these eleven are believed to be important toward maintaining good health in mammals.

These eleven saccharides include galactose, glucose, mannose, N-acetylneuraminic acid, fucose, N-acetylgalactosamine, N-acetylglucosamine and xylose (Murray et al., Harper's Biochemistry, 1996) as well as iduronic acid, arabinose and glucuronic acid, (Zubay et al., Principles of Biochemistry. Vol II, 1995). The structures of these carbohydrates are disclosed in Stryer's Biochemistry (Stryer, 1995) and the Merck Index, 12th Edition, 1996.

Recognizing this, scientists are currently trying, as yet with limited success, to synthetically attach glycosyl moieties to cytokines and other proteins. In fact, NIH has launched a project to develop methods to synthesize the glyco portion currently missing from their genetically engineered proteins. These synthetically produced cytokines have so far demonstrated disappointing results. Many challenges remain in this area. Scientists must first learn: 1) how to synthesize the glyco portion, 2) how to attach the glyco portion to the protein, and then 3) how to get the correct glycoproteins in the right concentrations to the right places in the body so as to facilitate good health.

For centuries, people of diverse cultures from around the world have utilized plants and herbs in the treatment of a wide variety of disorders in mammals. Specifically, formulations including poultices, teas, powders, pastes, extracts, plant or herb parts, plant or herbal extracts, lotions, creams, salves, troches, and others have been used. It is also now well recognized that much of the world's farm lands have been depleted of essential minerals required to sustain life, thus requiring the widespread use of vitamin, mineral and dietary supplements. A recent discovery concerns the importance of plant chemicals (phytochemicals) that are found in vine-ripened fruits and vegetables but are not found in those that are not vine-ripened. To provide these necessary, yet undefined, phytonutrients or phytonutritionals, as defined below, to the diet, some companies have begun supplying dietary supplements of freeze-dried, vine-ripened fruits and vegetables.

Nutritionists have developed hundreds of dietary supplement formulations in an effort to provide essential dietary components and facilitate and promote good health in mammals. However, fraudulent product claims regarding the treatment of physiological disorders are pervasive in the industry, and modern farming methods which focus on volume rather than nutritional value of crop production have led to crops having reduced dietary value that are missing essential dietary components.

Despite the extremely large number of dietary supplements available on store shelves today, the dietary needs of humans are still not being met. Many of such commercially available dietary supplements do not appear to provide any significant nutritional benefit. The present inventors believe such prior products suffer any one or more of the following disadvantages: a) they do not include the correct nutritional product(s); and b) their nutritional products are not well absorbed by a person taking them.

Thus, while scientists are beginning to recognize that other phytochemicals are required for good health, and others have previously recognized the utility of plants and herbs in the treatment of disorders, none of the known art suggests or discloses the invention as claimed herein. A need remains for non-pharmaceutical based dietary supplement formulations which provide essential saccharides that are the building blocks of glycoproteins and which promote good health in mammals.

DE-A-39 35 906 describes a nutritional composition to improve the anabolism of patients with metabolic deficits. The composition comprises at least 5 wt.% of the monosaccharide fraction consisting of compounds selected from the group, D-galactose, L-fucose, D-mannose, D-glucosamine, N-acetyl-D-galactosamine, N-acetylmannosamine, D-lactose and D-lactulose, alone or in mixtures thereof in which 50 mol% consists of D-galactose, its derivatives and precursors.

### SUMMARY OF THE INVENTION

It is an object of the present invention to provide a dietary supplement which promotes good health by providing to a mammal essential saccharides which are the building blocks of glycoproteins.

It has now been demonstrated herein by the present inventors that inclusion of these essential saccharides, as by supplementation of a diet with a dietary supplement containing the same, in the diets of mammals promotes good health. Although not intended to be limited to a particular mechanism of action, these essential saccharides are believed to be absorbed into the mammal's body and utilized in the formation of glycoproteins. By so providing these essential saccharides, the mammal's body does not have to spend energy unnecessarily catabolizing these essential saccharides and can therefore spend its energy providing for other physiological needs such as enhancement of the immune system to ward off, combat and/or ameliorate a wide range of physiological disorders.

Thus, the present invention overcomes the disadvantages and drawbacks of the prior art. One aspect of the present invention is directed to the use of various compositions of carbohydrates, i.e., glyconutritionals or glyconutrients, as dietary supplements which supplement a mammal's diet with sugars essential to glycoprotein and/or glycolipid production and thereby promote good health. In one embodiment, the present invention is directed to nutritional supplements including a defined amount of at least one of the eleven carbohydrates that are essential for the production of correctly structured and, therefore, properly functioning glycoproteins and/or glycolipids in a mammal. While some of these eleven sugars are readily available in common food sources, others are quite rare.

Accordingly, a first embodiment of the invention provides a dietary supplement for providing nutritional product saccharides which are essential components of glycoproteins in a mammal, said dietary supplement comprising a nutritionally effective amount of at least six saccharides, in monomeric, oligomeric or polymeric and derivatized or underivatized form, selected from the group consisting of:
galactose, glucose, mannose, N-acetylneuraminic acid, fucose, N-acetylgalactosamine, N-acetylglucosamine, xylose, arabinose, glucuronic acid, galacturonic acid, iduronic acid, arabinogalactan, acetylated mannose, glucosamine and galactosamine.

In other embodiments of the invention, the dietary supplement comprises nutritionally effective amounts of at least seven, at least eight, at least nine, at least ten or at least eleven saccharides, in monomeric, oligomeric or polymeric and derivatized or underivatized forms selected from the above listed group. Since some of these saccharides have ionizable groups, the invention contemplates all known non-toxic salt forms thereof.

The monomeric, oligomeric or polymeric and derivatized or underivatized forms of these saccharides can be obtained from a wide variety of sources, such as for example, gum tragacanth, guar gum, grain flour, rice flour, sugar cane, beet sugar, potato, milk, agar, algin, locust bean gum, psyllium, karaya gum, seed gums, Larch tree extract, aloe vera extract, gum ghatti, starch, cellulose, degraded cellulose, fructose, high fructose corn syrup, pectin, chitin, acacia, gum arabic, alginic acid, carrageenan, dextran, xanthan gum, chondroitin sulfate, sucrose, acetylated polymannose, maltose, glucan, lentinan, mannan, levan, hemi-cellulose, inulin, fructan, and lactose.

Other embodiments of the invention can comprise phytochemicals or phytonutritionals derived from ripened and freeze-dried fruits and vegetables, dioscorea complex, herbal extracts, herbal body-toning agents, beta sitosterol, melatonin, soy lecithin, vitamins, or minerals.

In another embodiment of the present invention, the compositions include predigested forms of at least one of the eleven essential carbohydrates. This can include one or all of the following: 1) physical digestion such as shearing or treatment with ultrasound, 2) chemical digestion such as enzymatic digestion, and acid or base hydrolysis, and 3) biological digestion with microbes such as bacteria, fungi or molds.

In another aspect, the present invention is a dietary supplement for the modification of behavior in alcohol dependent mammals comprising nutritionally effective amounts of the natural and/or synthetic monomeric, oligomeric and/or polymeric forms of acetylated mannose, gum ghatti, gum tragacanth, glucosamine, corn starch and arabinogalactan. In a particular embodiment, the dietary supplement will reduce the craving for alcohol in an alcohol dependent mammal being administered the supplement. In another particular embodiment, the dietary supplement will improve the overall well being of the alcohol dependent mammal by reducing at least one of depression and anger or increasing at least one of cognition, energy and positive outlook.

In yet another aspect, the present invention is a dietary supplement for the reduction of undesired side-effects in mammals receiving biologically effective agents that cause said side-effects, said dietary supplement comprising nutritionally effective amounts of the natural and/or synthetic monomeric, oligomeric and/or polymeric forms of acetylated mannose, gum ghatti, gum tragacanth, glucosamine, corn starch and arabinogalactan. In a particular embodiment, the dietary supplement will reduce the undesired side-effects of central nervous system drugs. In a more particular embodiment, the dietary supplement will reduce the undesired side-effects of methylphenidate in a mammal suffering from attention-deficit hyperactivity disorder and receiving methylphenidate.

### DETAILED DESCRIPTION

The body of a mammal hydrolyzes or metabolizes complex polysaccharides, such as plant carbohydrates, into various monosaccharides and subsequently forms oligosaccharides therefrom that are then used by the body to build the glycoproteins required by cytokines for cellular communication.

As used herein, the term "phytochemical" refers to plant synthesized molecules, found in food, or plant tissue in a complex organic matrix, which are minimally altered by processing from how they occur in nature. As used herein, the term "nutraceutical" refers to a non-toxic, nutrient of plant, mineral or animal origin, that has health promoting activity and that can be standardized and supplied as a dietary supplement to improve the nutritional quality of a balanced general diet. A nutraceutical is also a glyconutrient or phytonutrient.

As used herein, the terms "glyconutritional" or "glyconutrient" refer to complex carbohydrates or saccharides or simple sugars that are synthesized in nature and are necessary for the biochemical synthesis of various classes of communication and signal molecules that may be free in interstitial cellular fluids, active in cell to cell communication (i.e., cytokines, growth factors, etc.), or constitute the molecular configuration comprising foci of highly specific molecular activity of cell membranes (i.e., receptor sites, ion-transport channels, antigenic identification, and the like).

As used herein, the terms "phytonutritional" or "phytonutrient" refer to naturally synthesized molecules found only in plants that are produced to protect the plant's cells. Phytonutrients primarily have antioxidant, free-radical scavenger and vital micronutrient activity. These molecules, supplied through dietary supplementation, are found in mature plant tissues, and are most concentrated in seed coats and fruiting tissues surrounding the seed. In mammalian tissues, these molecules when supplied in the diet, are active in optimizing the biochemistry, immunology and physiology in the cellular micro-environment.

As used herein, the term "dioscorea complex" refers to an extract of dioscorea species (Mexican yam) providing a natural pre-cursor, dietary nutrient, diosgenin, a complex, six-ring, cyclic-carbon molecule that contains the molecular scaffold (perhydrocyclopentanophenanthrene) upon which mammalian adrenal and gonadal hormones are naturally synthesized. Providing this complex molecule in the diet can support optimal hormone balance, while maintaining normal physiological control mechanisms. This dietary supplement component has the potential to improve metabolic regulation of virtually every functioning cell in the body.

As used herein, the term "herbal extract" refers to phytochemicals that are produced in plant tissues and that can be extracted by water, polar, or petroleum solvents, and that have some degree of beneficial health or therapeutic activity. Most herbal agents can be toxic, especially when concentrated, but are generally safe when utilized in their more traditional manner in teas and poultices as a "folk medicinal for the treatment of disease and promotion of good health. As used herein, the term "herbal body-toning agent" refers to substances that have been observed by the inventors to reduce and reverse elastic tissue and collagen fiber damage caused by aging or sun-damage as evidenced by a restoration of skin turgor and elasticity which effectively reduces or eliminates wrinkles, sagging, hyperpigmentation and reversal of other undesirable elements of lost cosmetic appearance.

The carbohydrates included in the dietary supplement of the invention are available from a wide variety of natural and synthetic sources such as shrubs, trees, plants, yeasts, fungi, molds, gums, resins, starch and cellulose derivatives and natural mucin sources. Specifically, some of the natural sources include: (a) shrub or tree exudates which contain acacia, karaya, tragacanth, or ghatti; (b) marine gums which include agar, algin, or carrageenan; (c) seed gums which include guar, locust bean, or psyllium; (d) plant extracts which contain pectins or acetylated polymannose; (e) starch and cellulose derivatives such as hetastarch, carboxymethylcellulose, ethylcellulose, hydroxypropyl methylcellulose, methylcellulose, oxidized cellulose; and microbial gums which contain dextrans, xanthan. (Tyler et al., 1981) However, it should be recognized that the composition of the invention is not intended to be limited by the source from which the respective carbohydrates are obtained.

The saccharides of the invention can be found in nature as mono-, oligo- and/or polysaccharides. Thus, the compositions of the invention can contain the saccharides in their monomeric, oligomeric and/or polymeric forms. Table 3 below lists some of the known natural sources for the saccharides of the invention.

**Table 3.**

| **Natural sources of saccharides.** | |
|---|---|
| Source Carbohydrate | Corresponding Saccharide(s) |
| gum tragacanth | galacturonic acid galactose, fucose, xylose, arabinose and rhamnose |
| | |
| guar gum | mannose and galactose (1:2 molar ratio) |
| | |
| rice or grain flour | glucose |
| | |
| LAREX B-1000 | polyarabinogalactan |
| | |
| (Larch tree extract) | |
| | |
| MANAPOL™ | acetylated mannose based polymer |
| | |
| (aloe vera extract) | |
| | |
| gum ghatti | arabinose, galactose, mannose, xylose, glucuronic acid (10:6:2:1:2 molar ratio) |
| | |
| starch | glucose |
| | |
| pectin | galacturonic acid |
| | |
| chondroitin sulfate | N-acetylgalactosamine |
| | |
| chitin | N-acetylglucosamine |
| | |
| acacia, gum arabic | arabinose, galactose, glucuronic acid |
| | |
| alginic acid | mannosyluronic acid, gulosyluronic acid |
| | |
| carrageenan | galactose, 3,6-anhydrogalactose |
| | |
| dextran | glucose |
| | |
| xanthan gum | glucose, mannose, glucuronic acid |

It is well recognized in the art that the saccharides listed above with their corresponding source carbohydrates are present in particular amounts in nature as exemplified by the indicated molar ratios for the saccharides in gum ghatti and guar gum. The relative amounts or ratios of saccharides in natural carbohydrates is readily determined using conventional extraction or analytical methods or can be obtained from literature sources commonly used in the art.

As used herein, the term "carbohydrate" is used interchangeably with the terms "saccharide", "polysaccharide", "oligosaccharide" and "sugar" the definitions of which are well known in the art of carbohydrate chemistry. Although the compositions of the invention are intended to include at least one of the eleven essential saccharides, it should be noted that the saccharides can be in the form of mono-, oligo- and/or polysaccharides, e.g. a composition containing gum tragacanth and guar gum will be considered as containing galacturonic acid, fucose, xylose, arabinose and rhamnose, mannose and galactose. Therefore, by controlling the amount of particular gums in a given dietary supplement, one can control the amount of the respective saccharides in said dietary supplement.

Although the present invention includes the above cited eleven essential saccharides, it should be noted that other saccharides, nutritional compounds or biologically active or inert compounds can be included in the dietary supplement of the invention. Such other nutritional compounds include any one or more of phytonutrients, dioscorea complex, plant extracts, herbal extracts, plant parts, herbal components, vitamins or minerals. These nutritional compounds can be added to the dietary supplement of the invention, or they car be provided separately to a mammal being administered said dietary supplement. For example, a person receiving the glyconutrient-containing dosage form of the invention can also receive a phytonutrient in either the same or a separate dosage form. Inert compounds can include flavors, fillers, lubricants, buffers, gels, binders, excipients, carriers and/or other such compounds that facilitate the formulation or administration of the inventive dietary supplement. All of the glyconutrient-containing dietary supplement compositions of the invention, even those containing additional compounds, agents or other substances, can be obtained directly from MANNATECH™ (Coppell, TX).

Dioscorea complex is available from Ayusherbs (Japan) . When dioscorea complex is included in the dietary supplement of the invention, the ratio of dioscorea complex to total essential saccharide can range from about 0.0001/99.9999 to about 50/50 on a weight percent basis. In particular embodiments, the dioscorea complex to total essential saccharide ratio ranges from about 0.01-70/99.99-30 or about 10-40/90-60 or about 20/80.

Phytonutrients are available from a wide variety of manufacturing sources such as Cap-Tab (U.S.) or they can be added by freeze-drying and grinding ripe fruits and/or vegetables to form a powder which can then be added to or provided along with the dietary supplement of the invention. Such fruits and vegetables can be selected from all known fruits and vegetables but, in particular exemplary embodiments, include broccoli, brussel sprouts, cabbage, carrot, cauliflower, garlic, kale, onion, papaya, pineapple, tomato and turnip. These phytonutrients can be formulated in powder-containing caplet or capsule forms or in a base of gelatin and natural fruit fructose, optionally containing added flavors. When a phytonutrient is included in the dietary supplement of the invention, the ratio of total phytonutrient to total glyconutrient can range from about 0.001/99.999 to about 99.99/0.01 on a weight percent basis. As used herein, Phyto-1 refers to a dietary supplement comprising Glyco-1 (see Example 5), and freeze-dried raw fruits and vegetables. In particular embodiments, the phytonutrient to total glyconutrient ratio ranges from about 20-99/80-1 or about 50-95/50-5.

There are many plant and herbal extracts with suspected or demonstrated nutritional value which can promote good health and can be incorporated in or administered along with the dietary supplement of the invention. Such plant and herbal extracts can be obtained according to well known procedures for the extraction of substances, compounds or agents from plants or herbs. In particular embodiments, the dietary supplement of the present invention includes herbal or plant extracts of broccoli, brussel sprouts, cabbage, carrot, cauliflower, garlic, kale, onion, papaya, pineapple, tomato, asparagus, mushroom, parsnip, radish, and turnip. When a plant or herbal extract is included in the dietary supplement of the invention, the ratio of total extract (dry solids weight basis) to total glyconutrient can range from about 0.001-75/99.999-25 to about 10-90/90-10 on a weight percent basis.

Many different types of vitamins and minerals can be included in the dietary supplement of the invention. While a few vitamins and minerals of synthetic origin do possess nutritional value, particular embodiments of the dietary supplement herein contain nutritionally effective amounts of non-toxic vitamins and minerals obtained predominantly from natural sources. PROFILE™ is the tradename of a vitamin and mineral supplement used in the nutritional studies exemplified herein. This product, which can be obtained from MANNATECH™ (Coppell, TX), contains nutritionally effective amounts of the following vitamins and minerals: a) vitamins comprising A, B1 B12, B2, B6, beta carotene, bioflavanoids, biotin, C, choline, D, E, folic acid, inositol, K, niacinamide, para-aminobenzoic acid, and pantothenic acid; and b) minerals comprising boron, calcium, copper, GTF chromium, iodine, iron, magnesium, manganese, molybdenum, potassium, selenium, silicon, vanadium, and zinc. These vitamins and minerals may be provided in nutritionally acceptable non-toxic forms.

By "nutritionally effective amount" is meant that amount which will provide a beneficial nutritional effect or response in a mammal. For example, as nutritional response to vitamin- and mineral-containing dietary supplements varies from mammal to mammal, it should be understood that nutritionally effective amounts of said vitamins and minerals will vary, respectively. Thus, while one mammal may require a particular profile of vitamins and minerals present in defined amounts, another mammal may require the same particular profile of vitamins and minerals present in different defined amounts.

Other compounds, agents and nutrients can also be included in the dietary supplement of the invention, such as, for example, cellulose, calcium carbonate, kola nut, kola nut extract, country mallow, Atlantic kelp, cayenne pepper, silica, stearic acid, amino acids, glycine, lysine, glutamic acid, arginine, calcium carbonate, orchic substances, boron citrate, chromium picolinate, essential fibers, essential oils, essential botanicals, essential enteric ecology and flora growth promoters, essential fatty acids, live probiotic flora, proteins and enzymes.

The dietary supplement of the invention has been prepared and administered to mammals in powdered, reconstitutable powder, liquid-solid suspension, liquid, capsule, tablet, caplet, lotion and cream dosage forms. It should be readily obvious to one of ordinary skill in the science of formulations that the present dietary supplement can also be formulated appropriately for irrigation, ophthalmic, otic, rectal, sublingual, transdermal, buccal, vaginal, or dermal administration. Thus, other dosage forms such as chewable candy bar, concentrate, drops, elixir, emulsion, film, gel, granule, chewing gum, jelly, oil, paste, pastille, pellet, shampoo, rinse, soap, sponge, suppository, swab, syrup, chewable gelatin form, or chewable tablet can be used.

Due to varying diets among people, the dietary supplement of the invention can be administered in a wide range of dosages and formulated in a wide range of dosage unit strengths. For example, for those people who are missing from their diet nine of the eleven essential saccharides, a dietary supplement containing those nine saccharides in nutritionally effective amounts can be formulated. As well, for those people whose bioabsorption of essential saccharides is extremely efficient, a dietary supplement formulation containing reduced amounts of essential saccharides can be prepared.

It should be noted that the dosage of the dietary supplement can also vary according to a particular ailment or disorder that a mammal is suffering from when taking the supplement. For example, a person suffering from chronic fatigue syndrome, or fibromyalgia, will generally require a dose different than an alcoholic who is trying to discontinue alcohol consumption in order to obtain a nutritional benefit. An appropriate dose of the dietary supplement can be readily determined by monitoring patient response, i.e., general health, to particular doses of the supplement. As well, when another agent such as a phytonutrient, plant extract, herbal extract and/or dioscorea complex is being administered to a mammal along with the present glyconutritional dietary supplement, the appropriate doses of the supplement and each of the agents can be readily determined in a like fashion by monitoring patient response, i.e. general health, to particular doses of each.

It is contemplated by the invention that the dietary supplement can be administered simultaneously or sequentially in one or a combination of dosage forms. While it is possible and even likely that the present dietary supplement will provide an immediate overall health benefit, such benefit may take days, weeks or months to materialize. Nonetheless, the present glyconutritional dietary supplement will provide a beneficial nutritional response in a mammal consuming it.

It is also contemplated that the dietary supplement of the invention can be administered simultaneously or sequentially along with at least one of a phytonutrient, an herbal extract, a plant extract, and a dioscorea complex. Particular embodiments wherein the dietary supplement is administered simultaneously with at least one of a phytonutrient, an herbal extract, a plant extract, and a dioscorea complex are exemplified in the following examples.

For the examples herein, the dietary supplement of the invention was administered as a powder-containing capsule. When the dietary supplement included a phytonutrient, it was administered as a caplet or gelatin form. When the dietary supplement included a dioscorea complex, it was administered as either a capsule or caplet. When the dietary supplement included a phytonutrient, a dioscorea complex and an herbal extract, it was administered as a caplet.

According to the capsule or caplet size and ingredients used in a given study exemplified herein, the dietary supplement was administered initially as follows. The indicated doses are based upon #1 sized capsules and 1000 - 1200 mg caplets.

| SUPPLEMENT | DOSAGE |
|---|---|
| Glyco-1 | 2 capsules, 4x/day |
| | |
| Phyto-1 | 1 caplet, 4x/day |
| | |
| Glyco-1 with dioscorea complex | 1 caplet, 4x/day |
| | |
| PROFILE™ | 1 tablet, 3x/day |

As the exemplified studies proceeded, the doses of the supplements were modified according to patient response to a prior dosing regimen. For example, if a patient's overall health was not improving at the initial dose, the respective dose for one or more of the supplements was modified. It should be noted that the actual doses ultimately given to each patient in a study varied greatly from patient to patient as nutritional response varied. Generally, the dietary supplement and each of the other supplements was administered in the range of about 1 to about 12 capsules (or caplets or tablets) per day.

It is well documented that biochemical individuality exists among mammals and results in a very wide range of drug or food required to obtain a desirable health promoting effect. (Williams, R.; in Nutrition Against Disease, 1971). The amount of the above nutraceuticals typically utilized initially as a dietary supplement is indicated for conditions of compromised health. Energy level, stiffness, pain, discomfort, restful sleep, recovery from fatigue, and emotional status are used as nutritional benefit markers in determining a mammal's nutritional response to the dietary supplement and in determining whether or not an increase in dose is warranted. A reduction of health complaints or a reduction or elimination, of the above parameters is used as a guide for the reduction of glyconutrient intake. Complicating factors in regard to the amount of glyconutrients required for a benefit include the differing quantitative needs that individual have for nutrients, the differences being due to genetics, biochemical balance, disease state, altered physiology, prior and current general nutrition, individual choice and the nutrient content of food eaten by individuals. A desirable response or improvement in health is obtained when the missing nutrient or nutrients is/are adequately supplied by the present dietary supplement. The human body defends, repairs, regenerates, regulates, and heals itself through gene-control and nutrition provides the resources to accomplish these tasks. The inventive dietary supplement herein contain glyconutrients no longer commonly found in the urban/suburban food chain and thus supply a more optimal source of known and yet to be identified nutrients necessary for optimal biochemistry and physiology.

### EXAMPLE 1

A suitable composition for a product according to the present invention is as follows: tragacanth gum (100 kg), a source of galacturonic acid and galactose, fucose, xylose, arabinose and rhamnose is charged into a stainless steel ribbon blender and guar gum (10 kg), a source of mannose and galactose, is charged into the stainless steel ribbon blender. The mixture of tragacanth gum and guar gum is mixed for five (5) minutes. Then 250 grams of Aerosil 380™ (silica gel) is added to the mixture as a flowing agent and 200 kilograms of rice flour, a source of glucose, is added as a gluten-free filler. The mixture is then agitated for fifteen (15) minutes. Finally, 100 grams of calcium stearate is added to the mixture as a lubricant and the mixture is agitated for an additional three (3) minutes to generate a bulk powder. The powder is then encapsulated into size I gelatin capsules at a fill weight of 250 mg using a Model 8 (Elanco) capsule filling machine.

### EXAMPLE 2

A suitable composition for a product according to the present invention is as follows:

25 kilograms each of galactose, glucose, mannose, N-acetylneuraminic acid, fucose, N-acetylgalactosamine, N-acetylglucosamine, and xylose available from Florida Food Products as well as Aldrich Chemical Company and Sigma Chemical is charged into a stainless steel ribbon blender and mixed for five (5) minutes. Then 250 grams of Aerosil 380™ (silica gel) is added to the mixture as a flowing agent and 200 kilograms of rice flour, a source of glucose, is added as a gluten-free filler. The mixture is then agitated for fifteen (15) minutes. Finally, 100 grams of calcium stearate is added to the mixture as a lubricant and the mixture is agitated for an additional three (3) minutes to generate a bulk powder. The powder is then encapsulated into size #1 gelatin capsules at a fill weight of 250 mg using a Model 8 (Elanco) capsule filling machine.

### EXAMPLE 3

Another suitable composition for a bulk product according to the present invention is as follows. This formulation can be prepared according to Example 2. The weight percentages indicated are based upon the final weight of the composition.

| Percent by Weight | Ingredient | Approximate Density |
|---|---|---|
| 20 | Gum Tragacanth T/3 | 0.71 g/ml |
| | | |
| 20 | Gum Ghatti No. 1 | 0.79 g/ml |
| | | |
| 40 | arabinogalactan | 0.20 g/ml |
| | | |
| 20 | MANAPOL® | 0.12 g/ml |
| | combined ingredients | 0.30 g/ml |

Gum tragacanth T/3 and Gum Ghatti No. 1 are both tree exudates that are available from AEP Colloids of Ballston Spa, New York. Arabinogalactan is obtained from the Larch tree and is available from North American Pharmacal of Norwalk, Connecticut. MANAPOL® is a freeze-dried aloe vera extract available from Carrington Laboratories (Irving, Texas).

### EXAMPLE 4

### Standardization Assay

The following assay describes a method for standardization of concentrations of sugars covered by this patent.

*Standards:* All carbohydrate standards are available from Aldrich Chemical Company, Milwaukee, Wisconsin.

*Eluent:* Deionized (DI) water having a resistance greater than or equal to about 17 MOhm.

*Sample preparation:* 2 ml of 2 N hydrofluoric acid are added to 10mg of sample to be analyzed in a screw-top, TEFLON lined 10 ml test tube. The sample is then incubated at 120° C for one hour to hydrolyze into monosaccharides. The excess reagent is removed under a stream of air and the sample resuspended in 1 ml of DI water.

*HPLC Analysis:* AOAC Official Methods of Analysis 977.20

### EXAMPLE 5

The dietary supplement formulation of this example was prepared on large scale according to the above examples. This formulation, referred to as Glyco-1, includes the following ingredients in the amounts indicated. The weight percentage is based upon the weight of the final formulation containing all of the ingredients.

| Ingredient | Weight Percent |
|---|---|
| MANAPOL® | 10 |
| (aloe vera extract) | |
| gum ghatti | 10 |
| gum tragacanth | 10 |
| glucosamine | 10 |
| corn starch | 12 |
| arabinogalactan | 48 |

This composition was formulated into topical and oral preparations as indicated above.

### EXAMPLE 6

### Reduction of Medicine Induced Side-effects in the Treatment of Attention-Deficit Hyperactivity Disorder

Manual 4^{th} Ed. (DSM-IV) definitions for ADHD. One group consisted of five children whose parents had not placed them on methylphenidate (NO MED). The other 12 children in the study were receiving one of two different doses of methylphenidate: (a) six children received the normal prescribed dose (MED); and (b) six children received a reduced dose, i.e. below the normal prescribed dose (MED RED).

Assessment tools consisted of an ADHD rating scale for the DSM-IV symptoms; 18 items were rated on a scale of 0-3 for severity. Identical scales were constructed for the Oppositional Defiant Disorder (ODD) symptoms and the Conduct Disorder (CD) behaviors listed in DSM-IV. Both parents and teachers completed the above scales at each evaluation. In addition, parents completed a General Health Inventory for their children.

After all screening assessments were completed, all subjects had the glyconutritional product Glyco-1 added to their diets (1 capsule per 10 pounds of body weight for the first day and 1 capsule per 20 pounds of body weight for the remainder of the study). At week two, parent and teachers completed another rating series and the MED RED group had their medication reduced by half as per protocol. At week three, phytonutritionals (Phyto-1; 5 per day) were added to the dietary supplement procedure. The additional rating series were completed at weeks five and six.

The results indicated the Glyco-1 did not provide any further improvement in the ADHD symptomatology above that already obtained with the methylphenidate alone. However, a statistically significant reduction in the side-effects caused by the methylphenidate was obtained when Glyco-1 was administered to the subjects; therefore, an improvement in their overall general health was achieved.

### EXAMPLE 7

### Treatment of Alcoholics with Glyco-1

Glyco-1 capsules used in this study were prepared according to Example 6. The purpose of this study was to evaluate the effectiveness of dietary glyconutritional supplementation on the mood states and craving for alcohol in alcoholics. The study was conducted as follows.

Two groups of subjects were recruited from a local alcoholic support group in Little Rock, Arkansas: three recovering alcoholics and two practicing alcoholics. Each met the Diagnostic and Statistical Manual 4^{th} Ed. (DSM-IV) criteria for alcohol dependency. In the recovering group, abstinence varied from 2.5 years to six years and 11 months. For both groups, years of alcohol abuse ranged from 15 to 30 years and ages ranged from 33 to 62.

Assessment tools consisted of a self-rating scale of craving for alcohol which was scored from 0 to 9 and the Profile of Mood States (POMS). The POMS 65 items were divided into five scales: Cognitive, Depression, Energy, Anger/Temper, and Positive Outlook. These assessments were completed prior to taking glyconutritionals and again at the end of the five-week study.

Glyconutritionals were added to each subject's diet: 1 capsule per 10 pounds of body weight for the first day and thereafter 1 capsule per 20 pounds of body weight for the duration of the trial. No other interventions were introduced.

Results indicated that the mean initial alcohol craving of the five subjects had decreased in a statistically significant manner. Likewise, the results also indicated statistically significant improvements in the all of the measured mood states.

### EXAMPLE 8

### Treatment of various disorders with Glyconutrients

The following table summarizes the results obtained when patients were administered Glyco-1 either alone or in combination with one or more of Phyto-1, Glyco-1 with dioscorea and PROFILE™. Each patient was administered an initial dose Glyco-1 and any one or more of the respective supplements in the dosages indicated as follows:

| SUPPLEMENT | DOSAGE |
|---|---|
| Glyco-1 (A) | 2 capsules, 4x/day |
| | |
| Phyto-1 (B) | 1 caplet. 4x/day |
| | |
| Glyco-1 with dioscorea complex (C) | 1 caplet, 4x/day |
| | |
| PROFILE™ (D) | 1 tablet, 3x/day |
| "E" indicates a topical hydrogel formulation comprising glyconutritionals "F" indicates an oral dietary supplement comprising glyconutritionals and herbal extracts. "E" indicates a topical hydrogel formulation comprising glyconutritionals "F" indicates an oral dietary supplement comprising glyconutritionals and herbal extracts. | |

During each study, patient progress and nutritional or overall health response to administration of a given dietary supplement regimen was monitored. For those patients not responding well to initial doses, their dosing regimen was altered and their progress monitored again. It should be noted that in each of the cases, the Glyco-1 at an appropriate dose provided nutritionally effective amounts of the essential saccharide(s) necessary to promote good overall health in a given patient. That is, the glyconutrient-containing dietary supplement of the invention is not intended or professed to cure any of the disorders listed below. Rather, the dietary supplement provides a patient the necessary glyconutrients to permit a patient's own body to heal itself.

**Table 4.**

| **Disorders treated by administration of glyconutrients alone or in combination with one or move of phytonutrients, dioscorea complex and vitamins and minerals.** | | |
|---|---|---|
| DISORDER | NUTRITIONAL PRODUCTS ADMINISTERED | TREATMENT RESULTS |
| aging process or optimal health plan | A, B, C, D | decreased body fat; increased muscle mass and bone density; serum biochemistry altered to more healthy values |
| | | |
| old stable strokes | A, B, C | restored sensory and muscular control |
| | | |
| multiple sclerosis | A, B, C | restored sensory and muscular control |
| | | |
| amyotrophic lateral sclerosis | A, B, C | restored sensory and muscular control |
| | | |
| muscular dystrophy | A, B, C | restored sensory and muscular control |
| | | |
| cerebral palsy | A, B, C | restored sensory and muscular control |
| | | |
| macular degeneration | A, B, C | sight restorations |
| | | |
| seizures | A, B, C | reduction or elimination of allergies and infections; coordination, learning, memory and appearance improvements |
| | | |
| Down's Syndrome | A, B, C | reduction or elimination of allergies & infections; coordination, learning, memory and appearance improvements |
| | | |
| systemic combined immune deficiency syndrome | A, B, C | antibody and T-cell function restoration |
| | | |
| Tay-Sachs | A, B, C | restoration of lost functions |
| | | |
| retinitis pigmentosis | A, B, C | sight restoration |
| | | |
| color blindness | A, B, C | can see color |
| | | |
| Huntington's chorea | A, B, C | restoration or improvement of lost functions |
| | | |
| Alzheimer's | A, B, C | restoration or improvement of lost functions |
| | | |
| Parkinson's | A, B, C | restoration or improvement of lost functions |
| | | |
| inflammatory polyneuropathy | A, B, C | restoration or improvement of lost functions |
| | | |
| Closed head traumatic syndromes | A, B, C | restoration or improvement of lost functions |
| | | |
| spinal cord injury | A, B, C | restoration or improvement of lost functions |
| | | |
| ulcerative colitis | A, B, C | healed ulcers |
| | | |
| Crohn's disease | A, B, C | healed ulcers |
| | | |
| schizophrenia | A, B, C | improvements in functions |
| | | |
| depression | A, B, C | improvements in functions |
| | | |
| anxiety reactions | A, B, C | improvements in functions |
| | | |
| compulsive disorders | A, B, C | improvements in functions |
| | | |
| nervous tics | A, B, C | improvements in functions |
| | | |
| restless leg syndrome | A, B, C | improvements in functions |
| | | |
| Tourette's syndrome | A, B, C | improvements in functions |
| | | |
| autism | A, B, C | improvements in functions |
| | | |
| Wegener's granulomatosis | A, B, C | restoration of tissue |
| | | |
| Lupus E. | A, B | healing of lesions |
| | | |
| Rheumatoid arthritis | A, B | relief of symptoms |
| | | |
| thyroiditis | A, B | normalization of antinuclear antibodies |
| | | |
| myesthenia gravis | A, B | normalization of antinuclear antibodies |
| | | |
| diabetes mellitus | A, B | normalization of glucose and Hgb AIC; restoration of renal functions; healing of ulcers, elimination of infection; elevated lipids normalize; reduced insulin and glycomeds |
| | | |
| osteoporosis | A, B | reduced pain increased bone density |
| | | |
| alcoholism | A | reduction in craving |
| | | |
| cocaine | A | reduction in craving |
| | | |
| atherosclerosis | A, B | reduced total cholesterol, LDL, and triglycerides and increased HDL; improved patency of vessels and arrhythmia |
| | | |
| idiopathic myocarditis (presumed viral origin) | A, B | increased ejection function; restoration of heart size; increased Coxsackievirus antibody levels; and reversal of heart failure |
| | | |
| rheumatoid arthritis | A, B | elimination of pain, stiffness, fever, and swelling; restoration of scope of motion, strength and endurance |
| | | |
| degenerative arthritis | A, B | elimination of pain, stiffness, fever, and swelling; restoration of scope of motion, strength and endurance |
| | | |
| traumatic arthritis | A, B | elimination of pain, stiffness, fever, and swelling; restoration of scope of motion, strength and endurance |
| | | |
| juvenile arthritis | A, B | elimination of pain, stiffness, fever, and swelling; restoration of scope of motion, strength and endurance |
| | | |
| asthma | A | elimination of shortness of breath and wheezing and improvement of pulmonary function |
| | | |
| allergy - nasal, eyes, hay fever | A | elimination of itching, swelling, rash discomfort |
| | | |
| silicon breast implant | A, B, C | reduction or elimination of symptoms |
| | | |
| environmental toxin syndrome | A, B, C | reduction or elimination of symptoms |
| | | |
| agent orange | A, B, C | reduction or elimination of symptoms |
| | | |
| Gulf War syndrome | A, B, C | reduction or elimination of symptoms |
| | | |
| Hepatitis B & C | A, C, D | normalization of liver enzymes and symptoms |
| | | |
| influenza virus | A, C, D | prevention or amelioration; improvement of symptoms |
| | | |
| common cold | A, C, D | prevention or amelioration; improvement of symptoms |
| | | |
| AIDS | A, C, D | elimination of symptoms; m-RNA of HIV-1 is undetected; restored immune function |
| | | |
| herpes | A, C, D | elimination of infestations |
| | | |
| warts | A, C, D | elimination of infestations |
| | | |
| human papillovirus | A, C, D | elimination of infestations |
| | | |
| otitis media (chronic or persistent) | A, C, D | elimination of symptoms and need for antibiotics |
| | | |
| leukemia | A, B, C, D | correction of altered chromosomes |
| | | |
| lymphomas | A, B, C, D | normalization of tissue biopsies |
| | | |
| sarcomas (astrocytomas) | A, B, C, D | normalization of tissue biopsies |
| | | |
| adenocarcinomas such as breast, prostate, ovarian, gastrointestinal and lung | A, B, C, D | elimination of metastasis and shrinkage of mass to undetectable level |
| | | |
| profound introversion and female impotence | A, B, C, D | restoration of psychological interest and physiological sexual function in the elderly |
| | | |
| pain, ulcers and coldness of extremities in diabetes, raynauds, frost-bite, snake-bite and atherosclerosis | A, C, E | restoration to intact, painless extremity and microvascular circulation |
| | | |
| sun damaged skin, age damaged skin, and radiation damaged skin | A, C, E | lessening of pigmentation, wrinkles, and lost elasticity and restoration of dermis and epidermis |
| | | |
| athletic performance | C, F | increased strength and endurance, delayed fatigue, facilitation of recovery in young and aging athletes |

In summary, this invention pertains to the field of dietary supplements and nutritional support for promotion and maintenance of optimal good health. More specifically, the invention relates to compositions of carbohydrates as dietary supplements that are essential for the production of correctly structured and, therefore, properly functioning glycoproteins.

Science has recently shown that glycoproteins play a key role in all cellular communication. Many of the cytokines, i.e. cellular "words," do not function properly without an attached glycosyl moiety. The body hydrolyzes complex polysaccharides such as plant carbohydrates into various monosugars and restructures them into oligosaccharides that are then used by the body to build the glycoproteins required by cytokines for cellular communication and, thus, for good health.

This invention will correct the problem caused by modern diets consisting of highly refined foods, from which many essential ingredients have been eliminated during processing, specifically sugars needed for correctly structured and properly functioning glycoproteins.

The above is a detailed description of particular embodiments of the invention. Those of skill in the art should, in light of the present disclosure, appreciate that obvious modifications of the embodiments disclosed herein can be made without departing from the scope of the invention. All of the embodiments disclosed herein can be made and executed without undue experimentation in light of the present disclosure.

As used herein and unless otherwise indicated, the terms "a" and "an" are taken to mean "one", "at least one" or "one or more".

## Claims

1. A dietary supplement for providing nutritional product saccharides in monomeric. oligomeric or polymeric and derivatized or underivatized form, which saccharides are essential components of glycoproteins in a mammal, said dietary supplement comprising nutritionally effective amounts of:
at least six saccharides selected from a group of saccharides consisting of:
galactose, glucose, mannose, N-acetylneuraminic acid, fucose, N-acetylgalactosamine, N-acetylglucosamine, xylose, arabinose, glucuronic acid, galacturonic acid, iduronic acid, arabinogalactan, acetylated mannose, glucosamine and galactosamine.

2. A dietary supplement according to claim 1 comprising nutritionally effective amounts of at least seven saccharides selected from the group of saccharides.

3. A dietary supplement according to claim 1 comprising nutritionally effective amounts of at least eight saccharides selected from the . group of saccharides.

4. A dietary supplement according to claim 1 comprising nutritionally effective amounts of:
galactose, glucose, mannose, N-acetylneuraminic acid, fucose, xylose, arabinose, glucuronic acid, iduronic acid, N-acetylgalactosamine, and N-acetylglucosamine.

5. A dietary supplement according to claim 1 comprising nutritionally effective amounts of the natural and/or synthetic monomeric, oligomeric and/or polymeric forms of acetylated mannose, gum ghatti, gum tragacanth, glucosamine, corn starch and arabinogalactan.

6. A dietary supplement according to any one of claims 1-4, wherein said saccharides are provided in oligomeric or polymeric form as found in at least one of:
gum tragacanth, guar gum, grain flour, rice flour, sugar cane, beet sugar, potato, milk, agar, algin, locust bean gum, psyllium, karaya gum, seed gums, Larch tree extract, aloe vera extract, gum ghatti, starch, cellulose, degraded cellulose, fructose, high fructose corn syrup, pectin, chitin, acacia, gum arabic, alginic acid, carrageenan, dextran, xanthan gum, chondroitin sulfate, sucrose, acetylated polymannose, maltose, glucan, lentinan, mannan, levan, hemi-cellulose, inulin, fructan, and lactose.

7. A dietary supplement according to any one of claims 1-4 further comprising a nutritionally effective amount of dioscorea complex.

8. A dietary supplement according to any one of claims 1-4 further comprising a nutritionally effective amount of a blend consisting of ripened and freeze-dried and powdered raw fruits and vegetables.

9. A dietary supplement according to claim 8 further comprising a nutritionally effective amount of xanthines, and/or herbal body-toning agents.

10. A dietary supplement according to claim 8, wherein said blend consisting of ripened and freeze-dried and powdered raw fruits and vegetables comprises:
broccoli, brussel sprouts, cabbage, carrot, cauliflower, garlic. kale, onion, papaya, pineapple, tomato and turnip.

11. A dietary supplement according to claim 7 further comprising a nutritionally effective amount of a beta sitosterol.

12. A dietary supplement according to any one of claims 1-4 further comprising a nutritionally effective amount of melatonin.

13. A dietary supplement according to any one of claims 1-4 further comprising a nutritionally effective amount of a saccharide bioabsorption aid.

14. A dietary supplement according to claim 13, wherein said saccharide bioabsorption aid comprises soy lecithin.

15. A dietary supplement according to any one of claims 1-4 further comprising nutritionally effective amounts of a dioscorea complex and a blend consisting of ripened and freeze-dried and powdered raw fruits and vegetables.

16. A dietary supplement according to any one of claims 1-4 further comprising nutritionally effective amounts of non-toxic vitamins and minerals.

17. A dietary supplement according to claim 16, wherein
said vitamins comprise A, B1, B12, B2, B6, beta carotene, bioflavanoids, biotin, C, choline, D, E, folic acid, inositol, K, niacinamide, para-aminobenzoic acid, and pantothenic acid; and
said minerals comprise boron, calcium, copper, GTF, chromium, iodine, iron, magnesium, manganese, molybdenum, potassium, selenium, silicon, vanadium and zinc.

18. A dietary supplement according to any one of the preceding claims comprising a nutritionally effective amount of acetylated mannose.

## Patentansprüche

1. Nahrungsergänzungsmittel zur Bereitstellung von Sacchariden für Ernährungsprodukte in monomerer, oligomerer oder polymerer und derivatisierter oder underivatisierter Form, worin die Saccharide essentielle Komponenten von Glycoproteinen in einem Säugetier sind, wobei das Nahrungsergänzungmittel ernährungswirksame Mengen von wenigstens sechs Sacchariden umfaßt, ausgewählt aus einer Gruppe von Sacchariden, die aus Galactose, Glucose, Mannose, N-Acetylneuraminsäure, Fucose, N-Acetylgalactosamin, N-Acetylglucosamin, Xylose, Arabinose, Glucuronsäure, Galacturonsäure, Iduronsäure, Arabinogalactan, acetylierter Mannose, Glucosamin und Galactosamin besteht.

2. Nahrungsergänzungsmittel gemäß Anspruch 1, das ernährungswirksame Mengen von wenigstens sieben Sacchariden umfaßt, ausgewählt aus der Gruppe von Sacchariden.

3. Nahrungsergänzungsmittel gemäß Anspruch 1, das ernährungswirksame Mengen von wenigstens acht Sacchariden umfaßt, ausgewählt aus der Gruppe von Sacchariden.

4. Nahrungsergänzungsmittel gemäß Anspruch 1, das ernährungswirksame Mengen von Galactose, Glucose, Mannose, N-Acetylneuraminsäure, Fucose, Xylose, Arabinose, Glucoronsäure, Iduronsäure, N-Acetylgalactosamin und N-Acetylglucosamin umfaßt.

5. Nahrungsergänzungsmittel gemäß Anspruch 1, das ernährungswirksame Mengen der natürlichen und/oder synthetischen monomeren, oligomeren und/oder polymeren Formen von acetylierter Mannose, Ghatti-Gummi, Tragacanth-Harz, Glucosamin, Maisstärke und Arabinogalactan umfaßt.

6. Nahrungsergänzungsmittel gemäß einem der Ansprüche 1 bis 4, worin die Saccharide in oligomerer oder polymerer Form bereitgestellt sind, wie sie in wenigstens einem aus den folgenden gefunden werden: Tragacanth-Harz, Guar-Gummi, Getreidemehl, Reismehl, Zuckerrohr, Rübenzucker, Kartoffel, Milch, Agar, Algin, Johannisbrotkernmehl, Flohsamen, Karaya-Gummi, Samen-Gummen, Lärchen-Extrakt, Aloe vera-Extrat, Ghatti-Gummi, Stärke, Cellulose, abgebaute Cellulose, Fructose, Maissirup mit hohem Fructoseanteil, Pectin, Chitin, Akazie, Gummi arabicum, Alginsäure, Carrageenan, Dextran, Xanthan-Gummi, Chondroitinsulfat, Saccharose, acetylierte Polymannose, Maltose, Glucan, Lentinan, Mannan, Levan, Hemicellulose, Inulin, Fructan und Lactose.

7. Nahrungsergänzungsmittel gemäß einem der Ansprüche 1 bis 4, das ferner eine ernährungswirksame Menge von Dioscorea-Komplex umfaßt.

8. Nahrungsergänzungsmittel gemäß einem der Ansprüche 1 bis 4, das ferner eine ernährungswirksame Menge einer Mischung umfaßt, die aus gereiften und gefriergetrockneten und gepulverten rohen Früchten und Gemüsen besteht.

9. Nahrungsergänzungsmittel gemäß Anspruch 8, das ferner eine ernährungswirksame Menge von Xanthinen und/oder Kräuter-Körpertönungsmitteln umfaßt.

10. Nahrungsergänzungsmittel gemäß Anspruch 8, worin die Mischung, die aus gereiften und gefriergetrockneten und gepulverten rohen Früchten und Gemüsen besteht, Brokkoli, Rosenkohl, Kohl, Karotte, Blumenkohl, Knoblauch, Grünkohl, Zwiebel, Papaya, Ananas, Tomate und Rübe umfaßt.

11. Nahrungsergänzungsmittel gemäß Anspruch 7, das ferner eine ernährungswirksame Menge eines beta-Sitosterols umfaßt.

12. Nahrungsergänzungsmittel gemäß einem der Ansprüche 1 bis 4, das ferner eine ernährungswirksame Menge Melatonin umfaßt.

13. Nahrungsergänzungsmittel gemäß einem der Ansprüche 1 bis 4, das ferner eine ernährungswirksame Menge einer Saccharid-Bioabsorptionshilfe umfaßt.

14. Nahrungsergänzungsmittel gemäß Anspruch 13, worin die Saccharid-Bioabsorptionshilfe Sojalecithin umfaßt.

15. Nahrungsergänzungsmittel gemäß einem der Ansprüche 1 bis 4, das ferner ernährungswirksame Mengen eine Dioscorea-Komplexes und einer Mischung, die aus gereiften und gefriergetrockneten und gepulverten rohen Früchten und Gemüsen besteht, umfaßt.

16. Nahrungsergänzungsmittel einem der Ansprüche 1 bis 4, das ferner ernährungswirksame Mengen nicht-toxischer Vitamine und Mineralien umfaßt.

17. Nahrungsergänzungsmittel gemäß Anspruch 16, worin die Vitamine die folgenden umfassen: A, B1, B12, B2, B6, beta-Carotin, Bioflavanoide, Biotin, C, Cholin, D, E, Folsäure, Inosit, K, Niacinamid, para-Aminobenzoesäure und Pantothensäure; und worin die Mineralien die folgenden umfassen: Bor, Calcium, Kupfer, GTF, Chrom, Iod, Eisen, Magnesium, Mangan, Molybdän, Kalium, Selen, Silicium, Vanadium und Zink.

18. Nahrungsergänzungsmittel gemäß einem der vorhergehenden Ansprüche, das eine ernährungswirksame Menge von acetylierter Mannose umfaßt.

## Revendications

1. Complément alimentaire destiné à apporter le produit nutritionnel saccharides sous forme monomère, oligomère ou polymère, dérivée ou non dérivée, lesdits saccharides étant les composants essentiels des glycoprotéines chez un mammifère, ledit complément alimentaire contenant des quantités efficaces sur le plan nutritionnel d'au moins six saccharides choisis dans un groupe de saccharides constitué du galactose, du glucose, du mannose, de l'acide N-acétylneuraminique, du fucose, de la N-acétylgalactosamine, de la N-acétylglucosamine, du xylose, de l'arabinose, de l'acide glucuronique, de l'acide galacturonique, de l'acide iduronique, de l'arabinogalactane, du mannose acétylé, de la glucosamine et de la galactosamine.

2. Complément alimentaire selon la revendication 1, contenant des quantités efficaces sur le plan nutritionnel d'au moins sept saccharides choisis dans le groupe de saccharides.

3. Complément alimentaire selon la revendication 1, contenant des quantités efficaces sur le plan nutritionnel d'au moins huit saccharides choisis dans le groupe de saccharides.

4. Complément alimentaire selon la revendication 1, contenant des quantités efficaces sur le plan nutritionnel de galactose, de glucose, de mannose, d'acide N-acétylneuraminique, de fucose, de xylose, d'arabinose, d'acide glucuronique, d'acide iduronique, de N-acétylgalactosamine et de N-acétylglucosamine.

5. Complément alimentaire selon la revendication 1, contenant des quantités efficaces sur le plan nutritionnel des formes monomères, oligomères et/ou polymères, naturelles et/ou synthétiques, de mannose acétylé, de ghatti, de gomme adragante, de glucosamine, d'amidon de maïs et d'arabinogalactane.

6. Complément alimentaire selon l'une quelconque des revendications 1 à 4, dans lequel lesdits saccharides sont fournis sous forme oligomère ou polymère tels qu'ils se présentent dans au moins un parmi la gomme adragante, la gomme de guar, la farine de céréales, la farine de riz, la canne à sucre, le sucre de betterave, les pommes de terre, le lait, l'agar-agar, l'algine, la gomme de caroube, le psyllium, la gomme karaya, les gommes de grains, l'extrait de mélèze, l'extrait d'Aloe Vera, le ghatti, l'amidon, la cellulose, la cellulose dégradée, le fructose, le sirop de maïs à forte teneur en fructose, la pectine, la chitine, l'acacia, la gomme arabique, l'acide alginique, la carragénine, le dextran, la gomme de xanthane, le sulfate de chondroïtine, le saccharose, le polymannose acétylé, le maltose, le glucane, le lentinane, le mannane, le levane, l'hémicellulose, l'inuline, le fructane et le lactose.

7. Complément alimentaire selon l'une quelconque des revendications 1 à 4, contenant en outre une quantité efficace sur le plan nutritionnel d'un complexe d'igname.

8. Complément alimentaire selon l'une quelconque des revendications 1 à 4, contenant en outre une quantité efficace sur le plan nutritionnel d'un mélange constitué de fruits et de légumes crus, mûrs, lyophilisés et réduits en poudre.

9. Complément alimentaire selon la revendication 8, contenant en outre une quantité efficace sur le plan nutritionnel de xanthines et/ou d'agents tonifiants corporels à base d'herbes.

10. Complément alimentaire selon la revendication 8, dans lequel ledit mélange constitué de fruits et de légumes crus, mûrs, lyophilisés et réduits en poudre comprend des brocolis, des choux de Bruxelles, des choux, des carottes, des choux-fleurs, de l'ail, des choux frisés, des oignons, de la papaye, de l'ananas, des tomates et des navets.

11. Complément alimentaire selon la revendication 7, contenant en outre une quantité efficace sur le plan nutritionnel de β-sitostérol.

12. Complément alimentaire selon l'une quelconque des revendications 1 à 4, contenant en outre une quantité efficace sur le plan nutritionnel de mélatonine.

13. Complément alimentaire selon l'une quelconque des revendications 1 à 4, contenant en outre une quantité efficace sur le plan nutritionnel d'auxiliaire de bioabsorption de saccharide.

14. Complément alimentaire selon la revendication 13, dans lequel ledit auxiliaire de bioabsorption de saccharide contient de la lécithine de soja.

15. Complément alimentaire selon l'une quelconque des revendications 1 à 4, contenant en outre une quantité efficace sur le plan nutritionnel d'un complexe d'igname et d'un mélange constitué de fruits et de légumes crus, mûrs, lyophilisés et réduits en poudre.

16. Complément alimentaire selon l'une quelconque des revendications 1 à 4, contenant en outre une quantité efficace sur le plan nutritionnel de minéraux et de vitamines non toxiques.

17. Complément alimentaire selon la revendication 16, dans lequel lesdites vitamines comprennent les vitamines A, B1, B12, B2, B6, le β-carotène, les bioflavonoïdes, la biotine, la vitamine C, la choline, les vitamines D, E, l'acide folique, l'inositol, la vitamine K, le niacinamide, l'acide para-aminobenzoïque et l'acide pantothénique, et lesdits minéraux comprennent le bore, le calcium, le cuivre, le GTF, le chrome, l'iode, le fer, le magnésium, le manganèse, le molybdène, le potassium, le sélénium, le silicium, le vanadium et le zinc.

18. Complément alimentaire selon l'une quelconque des revendications précédentes, contenant une quantité efficace sur le plan nutritionnel de mannose acétylé.
